# EUROPEAN PATENT SPECIFICATION

(11) **EP 0 533 291 B1**
(45) Date of publication and mention of the grant of the patent: **04.12.1996**
(21) Application number: 92202857.6
(22) Date of filing: 17.09.1992
(51) Int. Cl.: C07C 69/54, C07C 67/38

(54) **Process for the preparation of methacrylate esters**
Verfahren zur Herstellung von Methacrylatestern
Procédé de préparation d'esters méthacryliques

(30) Priority: 20.09.1991 GB 9120122
(43) Date of publication of application: 24.03.1993
(73) Proprietor: SHELL INTERNATIONALE RESEARCH MAATSCHAPPIJ B.V., 2596 HR Den Haag (NL)
(72) Inventor: Hengeveld, Jan, NL-2596 HR The Hague (NL); de Blank, Peter Bastiaan, NL-2596 HR The Hague (NL)

(56) References cited:
- EP-A- 0 392 601

## Description

This invention relates to a continuous process for the preparation of methacrylate esters, wherein a mixture of C₃-hydrocarbons is drawn off from a refinery operation, a propyne stream is isolated from said C₃-mixture, and said propyne stream is fed to a carbonylation unit and contacted therein with carbon monoxide and an alcohol in the presence of a carbonylation catalyst to form a methacrylate ester.

Such a process, as described in EP-A-392,601, makes an economic use of readily available propyne-containing side streams from large scale industrial operations in the energy or petrochemical sector, and advantageously avoids the generation of large quantities of waste materials, such as sulphuric acid, ammonium bisulphate and toxic HCN.

These propyne-containing side streams will further contain propadiene, propane and propene as major constituents, of which propadiene can be used in the preparation of the methacrylates, as it is readily isomerized to propyne. The propane and propene components, however, will have to be removed, and are conveniently redirected to the original refinery operation for further processing or use. Accordingly, it is preferred that the methacrylate production is conducted in the proximity of and linked to the refinery operation providing the cheap propyne-containing side stream.

In practice, the refinery operations providing propyne-containing side streams involve multiple process and product lines and variable feedstocks, whereby the rate of propyne output is far from constant and subject to substantial fluctuations. Considering the commercial relationships, it will be clear that the methacrylate production is subordinate to the refinery operation. Therefore, the operation of the methacrylate production has to be adapted to the rate of the supply of the propyne-containing C₃-mixture, and thus to a fluctuating rate of propyne supply.

However, the design of a continuous carbonylation process operating at fluctuating flow rate appeared to be problematic in view of, inter alia, the cooling requirements of the exothermic reaction. A conceivable alternative solution of collecting the C₃-mixture in a buffer container, from which a stream could continuously be withdrawn at constant rate, was hampered by the requirement of redirecting the propane and propene components to the refinery operation and costs of the excessively large storage equipment. On the other hand, designing the buffer capacity to be placed after the step of isolation of the propyne component allowing undisturbed redirection of the propane and propene, entails the hazards of handling pure acetylenes. In order to comply with safety requirements, pure propyne should be maintained at temperatures below 5 °C, whilst a threatening calamity in case of an spontaneous temperature or pressure rise, has to be averted by discharge of the entire content of the storage container in view of the strong exotherm associated with the decomposition of propyne.

For overcoming the above problems, it is now proposed that a continuous process as mentioned in the preamble is conducted such, that at least part of the propyne stream and an alcohol feed stream is supplied to a storage facility, from which a combined propyne/alcohol feed is continuously withdrawn and introduced into the carbonylation unit.

The invention offers the advantages of rendering the production of methacrylate ester compatible with the refinery operation by simple means obviating or at least mitigating the requirements for handling pure propyne. The buffer of propyne supply can be maintained at temperatures up to ambient with reduced chance of any development of an exotherm. Should any uncontrolled rise of temperature and/or pressure occur, it will normally suffice to release the overpressure of the gaseous phase to maintain the propyne partial pressure within a safe range.

For benefiting by the advantages of the present invention, it is sufficient that part of the stream of isolated propyne is supplied to the storage facility, whilst the remainder is fed directly to the carbonylation unit, provided that the amount of propyne available in the storage facility is sufficient for supplementing the direct stream to the operational constant level at all times. For example, if a minimum propyne supply rate can be established for a given refinery operation, such a stream can be directly fed to the carbonylation unit, and any surplus be cut off and directed to the storage facility, from which a second constant stream is drawn off and supplied to the carbonylation unit. However, this mode may require substantial control.

For benefiting by the advantages of the present invention, it is sufficient that part of the alcohol feed is supplied to the storage facility whilst the remainder is fed directly to the carbonylation unit. This will mitigate the required safety measures for handling the buffer propyne. Alternatively, the entire alcohol feed may be supplied to the storage facility, whereby the maximum relief of safety measures is achieved. Again, such modes may require substantial control for ensuring appropriate stoichiometry and flow rate of the streams entering the carbonylation unit.

According to a very convenient, and preferred, embodiment of the invention, the entire propyne stream and alcohol feed stream are supplied to the storage facility. It is even more preferred, that substantially equimolar amounts of propyne and alcohol are supplied to the storage facility in accordance with the stoichiometry of the carbonylation reaction. In this way, a single feed stream can be withdrawn from the storage facility and supplied to the carbonylation unit at constant rate using a minimum of control devices. In the present context, "substantially equimolar" refers to molar ratios of propyne and alcohol being close to 1:1, as consumed in the overall process, notwithstanding a feed of minor amounts of one of the reactants, for example of the alcohol, as expedient in catalyst supply or recycle streams entering the carbonylation unit. Typically, "substantially equimolar" may represent ratios in the range Of from 0.8:1 to 1.2:1, more typically in the range of from 0.9:1 to 1.1:1.

The volume of the storage facility and the quantity of the propyne/alcohol mixture contained therein, can readily be chosen in relation to the scale of the process and the anticipated fluctuations of a particular propyne source. By way of example, for a process with a nominal output of 4000 kg/h of methyl methacrylate, a feed of averagely about 3032 kg/h of propyne/alcohol mixture is required at an effective propyne conversion of 95%, and a storage facility having a capacity of 72800 kg of an equimolar mixture of propyne and methanol would normally accommodate moderately high or low propyne supplies for a period of about 72 hours, or severely high or low supplies for a period of about 24 hours, as would seem to be appropriate when using a common propyne source, such as an ethene cracker.

The storage facility containing the buffer amount of propyne supply, is preferably maintained at a temperature in the range of from 0 to +20 °C, more preferably of from +5 to +15 °C. Such temperatures require very limited cooling utilities, and nevertheless secure safe operation conditions. The pressure within the storage facility is suitably maintained below 4.3 barabs, preferably below 3.7 barabs. To that end, the storage facility used in the present process is preferably provided with pressure release means operated to control the pressure to a maximum of 4.3 barabs.

In the context of the present invention, a "constant" rate is meant to define substantial constancy of the rate over a substantial period of time. It is intended to include both minor fluctuations of the rate within the accuracy of the control means of the process, and incidental or periodical adjustments of the actual value of the constant rate, for example a controlled increase or decrease of the rate, if the filling level of the storage facility reaches maximum or minimum capacity. Also, a "continuous" process is meant to mean a process proceeding without interruption over longer periods of time irrespective of incidental or periodical shut downs, e.g. for maintenance purposes.

Refinery operations particularly envisaged for supplying propyne-containing C₃-mixtures, include ethene crackers, i.e. a type of cracker in which ethene is prepared from hydrocarbon fractions such as naphtha, gas oil, LPG (preferably isobutane) or ethane by thermal cracking; catalytic crackers, i.e. a type of cracker in which hydrocarbons produced by the catalytic cracking of hydrocarbon fractions such as heavy gas oil or vacuum distillates; and LPG-dehydrogenation processes, i.e. a type of process in which propane is converted into propene, either thermally or catalytically. Such processes provide, amongst others, a C₃-stream consisting mainly of C₃-hydrocarbons, in particular propane, propene, propyne and propadiene. The refinery operation may be based on any carbon source, such as coal, crude or natural gas.

Methods for isolating propyne from C₃-mixtures are known, and, for example, described in EP-A-392 601. For example, the concentration of propyne and propadiene may be increased by selective scrubbing with a solvent, suitably being carried out in a column under elevated pressure (2-20, preferably 6-12 bar) using countercurrent flows of an organic solvent and the C₃-mixture (e.g. the bottom effluent of a propane/propene splitter), so that typically a stream consisting essentially of propane and propene (and < 0.2% of propyne/propadiene) is removed as the overhead fraction. The solvent which absorbs propyne and, at the elevated pressure employed, also propadiene, suitably comprises a polar organic solvent, such as an amide, e.g. dimethylformamide, dimethylacetamide or N-methylpyrrolidone, a nitrile, a sulphone, such as sulfolane, or a mixture thereof.

As propadiene tends to poison catalysts in the carbonylation of propyne and an alcohol, it is selectively removed, for instance by chemical means, such as by isomerization, and/or by physical means such as by distillation, preferably extractive distillation. Preferably, the propadiene is removed by physical means, and subsequently subjected to isomerization for partial conversion into propyne and recycle to the physical separation step. It will be appreciated that in this way all of the propyne and propadiene in the original C₃-mixture can in principle be isolated as propyne and used in the carbonylation reaction.

Extractive distillation is a well known method, in which the mixture of propyne and propadiene is dissolved in a polar organic solvent, or available as such a solution, and propadiene is removed as a gas (e.g. by stripping) leaving propyne dissolved in the solvent. Suitable solvents are the same as mentioned hereinabove. The isomerization of propadiene into propyne is well known in the art, and may conveniently be effected in the liquid or gas phase at a temperature in the range of from -30 to 100 °C, preferably 0 to 40 °C, and a pressure in the range of from 0.1 to 100 bar, preferably 1 to 20 bar, in the presence of an isomerization catalyst, for example a catalyst comprising an alkali metal or alkali metal oxide deposited on alumina, or another isomerization catalyst as described in Kirk-Othmer's Encyclopaedia of Chemical Technology, 2nd ed., Volume Supplement (1971), pages 547 to 556, and in US-A-3671605.

After selective removal of propadiene, substantially pure propyne can be isolated from the remaining solution, for example by stripping or flashing. The molar concentration in the propyne stream obtained lies above 80%, preferably above 90%, and most preferably above 99%. It is desirable that the molar ratio of propyne to propadiene in the propyne stream is above 100, preferably above 1000, and most preferably above 5000.

The carbonylation catalyst used in the process according to the invention may be any catalyst having activity for the carbonylation of propyne. It is preferably a Group VIII metal catalyst, more preferably a palladium catalyst. A preferred group of catalysts is disclosed in EP-A-186228, EP-A-271144 and EP-A-386833, and includes catalysts comprising a palladium compound, a ligand (e.g. a monodentate or bidentate phosphine) and an anion of a weakly or non-coordinating acid. Specific examples of such catalysts include combinations of (a) palladium acetate, (b) triphenylphosphine, diphenyl-2-pyridylphosphine, or diphenyl(6-methyl-2-pyridyl)phosphine, and (c) p-toluenesulphonic acid or trifluoroacetic acid.

The alcohol reacted in the carbonylation step of the present process can be any hydroxy-substituted hydrocarbon, which may or may not carry further substituents. The alcohol is preferably an alkanol having up to 20, more preferably 1 to 4 carbon atoms. Examples of alcohols are methanol, ethanol, propanol, isopropanol, n-butanol, isobutanol, and tert-butanol. Most preferably the alkanol is methanol, thus giving methyl methacrylate as the product.

The carbon monoxide reacted in the carbonylation step of the present process, can be derived from any source, and is preferably used in substantially pure form. Minor amounts of inerts or hydrogen may be present, but increase the need for bleed streams and purification of the end product, and therefore decrease the total yield of the present process, if present at too high concentration.

The reaction between propyne, the alcohol and carbon monoxide is preferably effected at a temperature in the range of from 20 to 200 °C, more preferably 20 to 80 °C, and at a pressure in the range of from 5 to 70 bar. Though a separate solvent may be used, for ease of purification it is preferably absent, since the alcohol precursor and methacrylate ester product constitute suitable liquid vehicles for the carbonylation reaction.

The reactants are preferably fed to the process in substantial accordance with the reaction stoichiometry, i.e. in substantially equimolar amounts apart from any recycle streams. The catalyst system is suitably applied at a concentration corresponding to its activity, for example, to produce 25 kg or more of methyl methacrylate per gram of palladium per hour.

The design and operation of the carbonylation unit and further work up equipment is within the skills of a chemical technologist, and does not require further explanation.

The storage facility used in the present process can be a container of any shape and magnitude, suitable for maintaining said liquid composition in quantities and for periods matching the design of the overall process. It will comprise inlets for the propyne and the alkanol stream and an outlet for the substantially equimolar mixture, and may further comprise flow monitoring and control means for steering the desired streams. Any construction material compatible with propyne and alcohols and the applied pressures, for example mild steel will be suitable.

As the propyne/alkanol mixture is preferably maintained at a temperature in the range of from 0 to +20 °C and a pressure below 4.3 barabs, the storage facility will comprise corresponding temperature and pressure control means, the latter preferably being a pressure release means operated at a desired maximum pressure, for example of 4.3 barabs.

## Claims

1. A continuous process for the preparation of methacrylate esters, wherein a mixture of C₃-hydrocarbons is drawn off from an oil or gas processing operation, a propyne stream is isolated from said C₃-mixture, and said propyne stream is fed to a carbonylation unit and contacted therein with carbon monoxide and an alcohol in the presence of a carbonylation catalyst to form a methacrylate ester, characterized in that at least part of the propyne stream and an alcohol feed stream is supplied to a storage facility, from which a combined propyne/alcohol feed is continuously withdrawn and introduced into the carbonylation unit.

2. A process as claimed in claim 1, wherein the entire propyne stream and alcohol feed stream are supplied to the storage facility.

3. A process as claimed in claim 1 or 2, wherein substantially equimolar amounts of propyne and alcohol are supplied to the storage facility.

4. A process as claimed in any one or more of claims 1-3, wherein said storage facility is maintained at a temperature in the range of from 0 to +20 °C, and a pressure of below 4.3 barabs.

5. A process as claimed in any one or more of claims 1-4, wherein the alcohol is an alkanol having 1-4 carbon atoms.

6. A process as claimed in any one or more of claims 1-5, wherein the combined propyne/alcohol feed is withdrawn from the storage facility at a constant rate.

7. A process according to any one of claims 1-6, wherein the storage facility contains a liquid composition consisting of substantially equimolar amounts of propyne and an alkanol having 1-4 carbon atoms.

8. A process as claimed in claim 7, wherein said liquid composition is contained at a temperature in the range of from 0 to +20 °C.

9. A process as claimed in claim 7 or 8, wherein the storage facility is provided with pressure release means.

10. A process as claimed in claim 9, wherein the pressure release means are set to a maximum pressure control of up to 4.3 barabs.

## Patentansprüche

1. Kontinuierliches Verfahren zur Herstellung von Methacrylsäureestern, bei dem man ein Gemisch aus C₃-Kohlenwasserstoffen aus einem Öl- oder Gasverarbeitungsverfahren abzieht, aus dem C₃-Gemisch einen Propinstrom isoliert, diesen einer Carbonylierungsanlage zuführt und ihn darin mit Kohlenmonoxid und einem Alkohol in Gegenwart eines Carbonylierungskatalysators zu einem Methacrylsäureester umsetzt, dadurch gekennzeichnet, daß man mindestens einen Teil des Propinstroms und einen Teil enines Alkoholeinsatzstroms einer Lagervorrichtung zuführt, aus der man kontinuierlich einen kombinierten Propin-Alkohol-Einsatz abzieht und in die Carbonylierungsanlage einspeist.

2. Verfahren nach Anspruch 1, bei dem man der Lagervorrichtung den gesamten Propinstrom und Alkoholeinsatzstrom zuführt.

3. Verfahren nach Anspruch 1 oder 2, bei dem man der Lagervorrichtung weitgehend äquimolare Mengen Propin und Alkohol zuführt.

4. Verfahren nach einem oder mehreren der Ansprüche 1-3, bei dem man die Lagervorrichtung bei einer Temperatur im Bereich von 0 bis +20°C und einem Druck von unter 4,3 bar abs. hält.

5. Verfahren nach einem oder mehreren der Ansprüche 1-4, bei dem man als Alkohol ein Alkanol mit 1-4 Kohlenstoffatomen einsetzt.

6. Verfahren nach einem oder mehreren der Ansprüche 1-5, bei dem man den kombinierten Propin-Alkohol-Einsatz mit konstanter Geschwindigkeit aus der Lagervorrichtung abzieht.

7. Verfahren nach einem der Ansprüche 1-6, wobei die Lagervorrichtung eine flüssige Zusammensetzung, die aus weitgehend äquimolaren Mengen an Propin und einem Alkanol mit 1-4 Kohlenstoffatomen besteht, enthält.

8. Verfahren nach Anspruch 7, bei dem man die flüssige Zusammensetzung bei einer Temperatur im Bereich von 0 bis +20°C lagert.

9. Verfahren nach Anspruch 7 oder 8, wobei die Lagervorrichtung mit Druckentlastungseinrichtungen versehen ist.

10. Verfahren nach Anspruch 9, bei dem man die Druckentlastungseinrichtungen auf einen Maximaldruck von bis zu 4,3 bar abs. einstellt.

## Revendications

1. Procédé continu de préparation d'esters méthacryliques, dans lequel un mélange d'hydrocarbures en C₃ est soutiré d'une opération de traitement de pétrole ou de gaz, un courant de propyne est isolé dudit mélange en C₃, et ledit courant de propyne est chargé dans une unité de carbonylation et y est mis en contact avec du monoxyde de carbone et un alcool, en présence d'un catalyseur de carbonylation en vue de former un ester méthacrylique, caractérisé en ce qu'au moins une partie du courant de propyne et un courant de charge d'alcool alimentent un dispositif de stockage, à partir duquel une charge combinée de propyne/alcool est soutirée en continu et introduite dans l'unité de carbonylation.

2. Procédé selon la revendication 1, dans lequel la totalité des courants de propyne et de charge d'alcool alimente le dispositif de stockage.

3. Procédé selon la revendication 1 ou 2, dans lequel des quantités sensiblement équimolaires de propyne et d'alcool alimentent le dispositif de stockage.

4. Procédé selon l'une quelconque ou plusieurs des revendications 1 à 3, dans lequel ledit dispositif de stockage est maintenu à une température dans le domaine de 0 à +20°C, et à une pression inférieure à 4,3 bars.

5. Procédé selon l'une quelconque ou plusieurs des revendications 1 à 4, dans lequel l'alcool est un alcanol ayant 1 à 4 atomes de carbone.

6. Procédé selon l'une quelconque ou plusieurs des revendications 1 à 5, dans lequel la charge combinée de propyne/alcool est soutirée à partir du dispositif de stockage à débit constant.

7. Procédé selon l'une quelconque des revendications 1 à 6, dans lequel le dispositif de stockage contient une composition liquide constituée de quantités sensiblement équimolaires de propyne et d'un alcanol ayant 1 à 4 atomes de carbone.

8. Procédé selon la revendication 7, dans lequel ladite composition liquide est maintenue à une température dans le domaine de 0 à +20°C.

9. Procédé selon la revendication 7 ou 8, dans lequel le dispositif de stockage est pourvu de moyens de relâchement de pression.

10. Procédé selon la revendication 9, dans lequel les moyens de relâchement de pression sont réglés à une limite de pression maximum allant jusqu'à 4,3 bars.
